# EUROPEAN PATENT SPECIFICATION

(11) **EP 4 106 838 B1**
(45) Date of publication and mention of the grant of the patent: **21.01.2026**
(21) Application number: 21712210.0
(22) Date of filing: 15.02.2021
(51) Int. Cl.: A61M 1/36, A61J 1/10, A61J 1/14, A61J 1/20

(54) **DEVICE FOR THE PRIMING OF AN EXTRACORPOREAL CIRCUIT**
VORRICHTUNG ZUM ENTLÜFTEN EINES EXTRAKORPORALEN KREISLAUFS
DISPOSITIF POUR L'AMORÇAGE D'UN CIRCUIT EXTRACORPOREL

(30) Priority: 18.02.2020 IT 202000003308
(43) Date of publication of application: 28.12.2022
(73) Proprietor: Eurosets S.r.l., 41036 Medolla (MO) (IT)
(72) Inventor: PETRALIA, Antonio, 41036 Medolla (MO) (IT); GHELLI, Nicola, 41036 Medolla (MO) (IT); FONTANILI, Paolo, 41036 Medolla (MO) (IT)
(74) Representative: Zoli, Filippo
(86) International application number: PCT/IB2021/051238
(87) International publication number: WO 2021/165810

(56) References cited:
- DE-A1- 4 208 054
- US-A1- 2010 049 115
- US-A1- 2017 296 735

## Description

### Technical Field

The present invention relates to a device for the priming of an extracorporeal circuit.

### Background Art

As is well known, in certain surgical operations, during which the functions of the patient's heart are temporarily interrupted, extracorporeal blood circuits are performed using the so-called "heart-lung" machines.

Heart-lung machines comprise a series of devices, including a filtering device (also known as a "venous reservoir") adapted to filter the incoming blood from the patient, a heat exchanger adapted to regulate the temperature of the blood leaving the filtering device, and an oxygenator adapted to provide the correct supply of oxygen to the blood intended to be pumped back into the patient. In particular, the incoming blood from the patient is sent to the oxygenator by means of a relevant pumping unit.

Before using the extracorporeal circuit it is necessary to carry out what is called, in jargon, the priming of the circuit itself, i.e. its filling by means of a liquid solution in order to remove the air inside.

This operation is generally carried out using a bag that is connected to the various devices of the extracorporeal circuit in order to define a closed circuit, in order to push the air contained inside the circuit itself to the outside. In particular, the bags of this type are provided with a first duct through which the operating liquid is introduced, a second duct adapted to let the liquid flow outwards from the bag in order to flow through the various devices that make up the extracorporeal circuit, a third duct adapted to reintroduce the operating liquid inside the bag, and a fourth duct adapted to allow the air to escape outwards.

In order to prevent the operating liquid from flowing through the circuit also in the opposite direction, a one-way valve is generally positioned outside the bag, along the pipes that carry the operating liquid to the bag itself.

This solution does however have some drawbacks.

In particular, the operating liquid that is introduced into the bag through the first duct can, at the same time, flow out both through the second duct and through the third duct, i.e. in two opposite directions. It follows, therefore, that part of the air contained inside the bag or the ducts connected thereto can be pushed towards the extracorporeal circuit, which must be properly de-bubbled rather than moving away therefrom.

It follows, therefore, that the devices used to date do not ensure effective de-bubbling of the extracorporeal circuit during the priming operations.

US 2017/296735A1 discloses a device for the priming of an extracorporeal circuit of the known type.

### Description of the Invention

The main aim of the present invention is to devise a device for the priming of an extracorporeal circuit to remove the air contained therein in a convenient and effective manner.

Within this aim, one object of the present invention is to devise a device that is simple to implement and of low cost, as well as safe to operate.

Another object of the present invention is to devise a device for the priming of an extracorporeal circuit that allows overcoming the aforementioned drawbacks of the prior art within a simple, rational, easy, effective to use as well as low cost solution.

The objects mentioned above are achieved by the present device for the priming of an extracorporeal circuit according to claim 1.

### Brief Description of the Drawings

Other characteristics and advantages of the present invention will become more evident from the description of a preferred, but not exclusive, embodiment of a device for the priming of an extracorporeal circuit, illustrated by way of an indicative, yet non-limiting example, in the accompanying tables of drawings wherein:

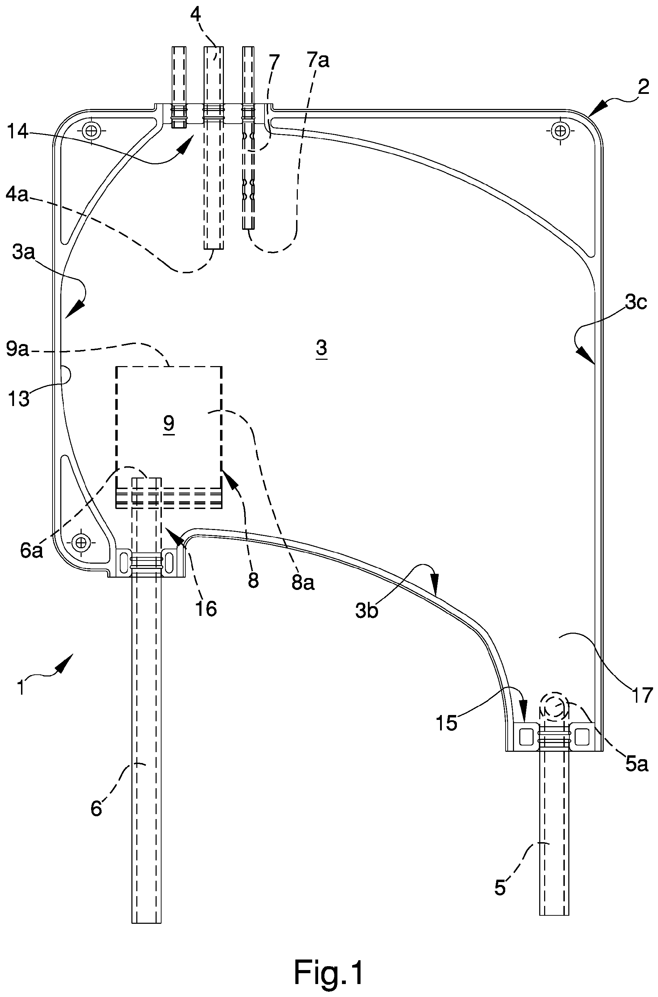

Figure 1 is a front elevation view of a device according to the invention in a first embodiment;
Figure 2 is a front elevation view of a device according to the invention in a
second embodiment.

### Embodiments of the Invention

With particular reference to these figures, reference numeral 1 globally indicates a device for the priming of an extracorporeal circuit.

The device 1 comprises a bag 2 made of flexible material and defining a containment volume 3 of a liquid and a plurality of ducts 4,5,6,7 associated therewith, of which at least a first duct 4, at least a second duct 5, at least a third duct 6 and at least a fourth duct 7, provided with a first port 4a, a second port 5a, a third port 6a and a fourth port 7a, respectively, arranged inside the containment volume 3.

In particular, the first port 4a is adapted to allow the introduction of an operating liquid inside the containment volume 3, the second port 5a is adapted to allow the outflow of the operating liquid to the outside, the third port 6a is adapted to reintroduce the operating liquid inside the containment volume 3 and the fourth port 7a allows the outflow of the air present in the containment volume itself and in the operating liquid to the outside.

In the embodiment shown in the figures, the first duct 4 is separate from the second duct 5 and the fourth duct 7.

It cannot however be ruled out that if the first duct 4 is separate from the second duct 5, it may still coincide with the fourth duct 7. In this case, not shown in the figures, the first port 4a through which the operating liquid is introduced inside the containment volume 3 coincides with the fourth port 7a through which the air present in the volume itself is conveyed to the outside. The aforementioned duct is therefore used, alternately, as a duct for the introduction of the operating liquid and as a duct for the outflow of air.

In a further embodiment, not shown in the figures, the first duct 4 coincides with the second duct 5. In this embodiment, the first port 4a coincides, therefore, with the second port 5a. In this case, a fitting, e.g. of the Y type or of the Luer Lock type, is provided along said duct for the introduction of the operating liquid inside the containment volume 3. Also in this case, the aforementioned duct is used, alternately, as a duct for the introduction of the operating liquid inside the containment volume 3 and as a duct for the outflow of the liquid from the containment volume itself.

The second duct 5 and the third duct 6 are connected to each other by interposition of the devices making up the extracorporeal circuit, such as, e.g., a centrifugal pump and an oxygenator. In particular, these devices are connected at inlet to the second duct 5 and at outlet to the third duct 6.

According to the invention, the device 1 comprises one-way valve means 8 associated with the third duct 6, at the point where the third port 6a is located, and adapted to prevent the flow of the operating liquid from the containment volume 3 to the third duct itself. Thus, the valve means 8 are only adapted to allow the operating liquid to flow from the third duct 6 to the containment volume 3 but not vice versa.

Preferably, the valve means 8 are shaped so as to define at least one containment chamber 9 of the third port 6a, wherein such a containment chamber 9 is provided with at least one closable opening 9a which is adapted to allow the transit of the operating liquid flowing out of the third port 6a inside the containment volume 3 but not vice versa.

The third port 6a is then arranged inside the containment chamber 9 defined by the valve means 8.

In other words, the flaps bordering the opening 9a approach each other so as to close the opening itself, thus preventing the flow of the operating liquid, when the latter is outside the containment chamber 9, while they move away from each other, thus defining the opening 9a so as to allow the flow of the operating liquid, when the latter is contained inside the containment chamber 9.

More particularly, the valve means 8 are of the type of a bag made of flexible material, which defines the containment chamber 9 and the opening 9a. The third port 6a is then enveloped by the bag defined by the valve means 8.

In a particular embodiment, the one-way valve means 8 comprise at least two sheets 8a made of a flexible material and sealed together along at least three sides contiguous to each other, through one of which the third duct 6 passes. The non-sealed side of the sheets 8a defines the closable opening 9a.

As can be seen from the figures, the opening 9a is substantially facing the third port 6a.

Advantageously, the device 1 further comprises de-bubbling means 10 of the air contained in the operating liquid placed inside the containment volume 3. Appropriately, the de-bubbling means 10 are placed around, i.e. surrounding, the one-way valve means 8.

In more detail, the de-bubbling means 10 define a de-bubbling chamber 11, the walls of which are permeable to air and liquids, inside which both the valve means 8 and the fourth port 7a are arranged.

The de-bubbling means 10 are made e.g. of a micro-perforated mesh adapted to facilitate the escape, through the fourth duct 7, of the air still present in the operating liquid that re-enters inside the containment volume 3 through the third duct 6.

For example, the bag 2 is made of two sheets of flexible material overlapping each other and sealed, or otherwise mutually made integral, so as to define the containment volume 3. The containment volume 3 is then bordered by a plurality of perimeter edges defined by the union of the two overlapping sheets. Preferably, the containment volume 3 has a maximum cross-section, detected in a transverse direction to the longitudinal extension of the ducts 4,5,6,7, at the point where the opening 9a is located.

In particular, the containment volume 3 is bordered by at least a first lateral edge 3a that defines a concavity 13 facing inwards the containment volume itself. In other words, the first lateral edge 3a is shaped so as to define an "overhang" facing outwards.

Appropriately, as visible from the figures, the first lateral edge 3a has an at least partly curved extension.

As can be seen from the figures, the first duct 4, the second duct 5 and the third duct 6 are positioned, respectively, at a first zone 14, at a second zone 15 and at a third zone 16 of the bag 2, wherein the first zone 14 is placed in the upper portion of the bag 2 and wherein the second zone 15 and the third zone 16 are placed in the lower portion of the bag itself. In turn, the fourth duct 7 is also placed at the point where the first zone 14 is located. The terms "upper" and "lower" used herein relate to the position in which the bag is commonly placed during its use.

More particularly, the first lateral edge 3a connects the first zone 14 and the third zone 16 to each other.

Advantageously, the second zone 15 is arranged at a lower level than the third zone 16 and so also the second port 5a is arranged at a lower level than the third port 6a.

Preferably, the containment volume 3 is also bordered by at least one substantially curved bottom edge 3b connecting the third zone 16 to the second zone 15. Since, as described above, the second zone 15 is arranged at a lower level than the third zone 16, the bottom edge 3b has a descending pattern as it proceeds close to the second zone 15.

The containment volume 3 is bordered, then, by a second lateral edge 3c, arranged at the point where the second zone 15 is located, on the opposite side of the first lateral edge 3a; the second lateral edge 3c and a section of the bottom edge 3b define a collecting column 17 of the operating liquid arranged on top of the second port 5a. The collecting column 17 has a significantly smaller cross section than the aforementioned maximum cross section.

The operation of the present invention is as follows.

After the extracorporeal circuit, and therefore the relevant devices that make it up, have been connected to the second and third ducts 5 and 6, the operating liquid is introduced inside the bag 2 through the first duct 4.

The operating liquid entering the inside of the containment volume 3 is conveyed as it progresses towards the second port 5a so as to flow through the second duct 5. More particularly, the operating liquid collects in the collecting column 17.

The valve means 8 prevent the operating liquid that is introduced inside the containment volume 3 from entering the inside of the third duct 6 through the third port 6a. More particularly, due to the presence of the operating liquid outside the containment chamber 9 the opening 9a closes, thus preventing the flow of the operating liquid itself.

The operating liquid then flows entirely through the second port 5a, entering the second duct 5, so as to reach the extracorporeal circuit and the relevant devices that make it up.

Once through the extracorporeal circuit, which is then filled with the operating liquid, the latter enters inside the third duct 6 re-entering inside the containment volume 3 through the third port 6a.

In more detail, the operating liquid flowing out of the third port 6a is initially contained inside the containment chamber 9 defined by the valve means 8 and then flows out thereof through the opening 9a, thereby flowing inside the containment volume 3.

The liquid that is returned to the inside of the containment volume 3 through the third duct 6 carries with it the air which was contained inside the ducts themselves and inside the extracorporeal circuit and which is expelled outwards through the fourth duct 7.

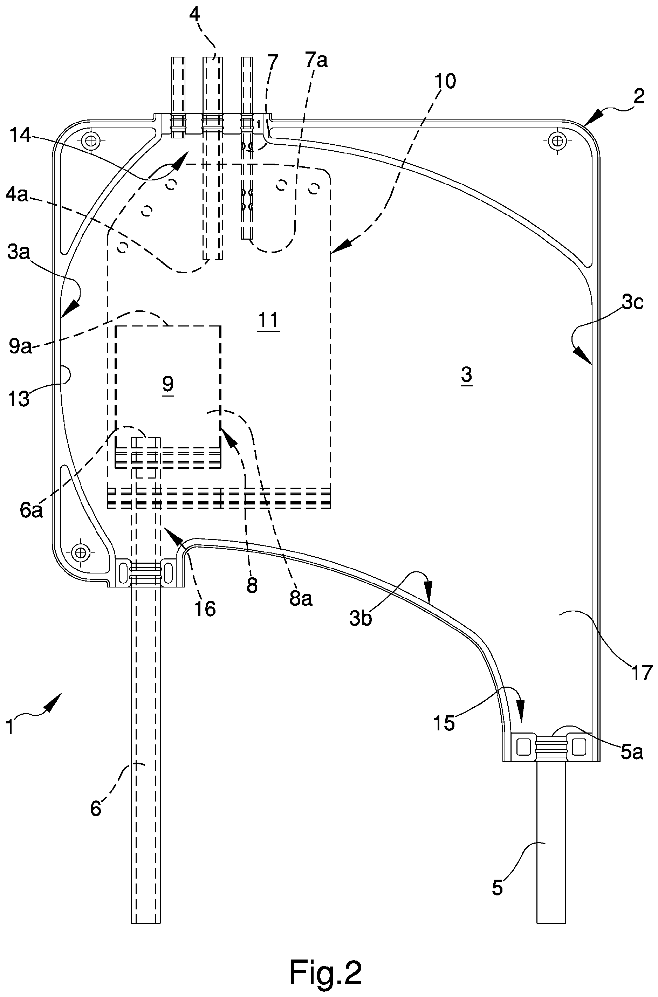

In the second embodiment shown in Figure 2, the operating liquid flowing out of the valve means 8 is contained inside the de-bubbling chamber 11 in order to facilitate the outflow of air, and in particular of micro-bubbles, through the fourth port 7a.

It has in practice been ascertained that the described invention achieves the intended objects and, in particular, it is emphasized that the device to which the present invention relates allows, thanks to the presence of the one-way valve means arranged to surround the third port, ensuring in an autonomous manner, i.e. without the aid of further devices, that the operating liquid follows the preferential path to remove the air contained in the extracorporeal circuit, thus avoiding its entry inside the third duct.

In addition, the presence of the de-bubbling means arranged to surround the one-way valve means facilitates the outflow of the micro-bubbles present in the operating liquid.

## Claims

1. Device (1) for the priming of an extracorporeal circuit, comprising:
- a bag (2) made of flexible material and defining a containment volume (3) of a liquid;
and at least three ducts used as:
- at least a first duct (4) associated with said bag (2) and provided with a first transit port (4a) placed inside said containment volume (3) for the introduction of an operating liquid inside it;
- at least a second duct (5) associated with said bag (2) and provided with a second transit port (5a) placed inside said containment volume (3) for the outflow of the operating liquid to the outside;
- at least a third duct (6) associated with said bag (2) and provided with a third transit port (6a) placed inside said containment volume (3) for the reintroduction of the operating liquid inside it;
- at least a fourth duct (7) associated with said bag (2) and provided with a fourth transit port (7a) placed inside said containment volume (3) for the outflow to the outside of the air present in the containment volume itself and in the operating liquid;
wherein the first, second, third, and fourth ducts (4, 5, 6, 7) are separate from one another, or
said first duct (4) coincides with said second duct (5), said first port (4a) coinciding with said second port (5a) or
said first duct (4) is separate from said second duct (5) and coincides with said fourth duct (7), said first port (4a) coinciding with said fourth port (7a);
**characterized by** the fact that it comprises one-way valve means (8) associated with said third duct (6), at the point where said third port (6a) is located, and adapted to prevent the transit of the operating liquid from said containment volume (3) to said third duct (6) and by the fact that said one-way valve means (8) define at least one containment chamber (9) of said third port (6a), said containment chamber (9) being provided with at least one closable opening (9a) adapted to allow the transit of the operating liquid flowing out of said third port (6a) inside said containment volume (3).

2. Device (1) according to claim 1, **characterized by** the fact that said one-way valve means (8) are of the type of a bag made of flexible material defining said containment chamber (9) and said closable opening (9a).

3. Device (1) according to claim 1 or 2, **characterized by** the fact that said one-way valve means (8) comprise at least two sheets made of flexible material and sealed together along at least three sides.

4. Device (1) according to claims 1 to 3, **characterized by** the fact that said closable opening (9a) is substantially facing said third port (6a).

5. Device (1) according to one or more of the preceding claims, **characterized by** the fact that it comprises de-bubbling means (10) of the air contained in the operating liquid placed inside said containment volume (3).

6. Device (1) according to claim 5, **characterized by** the fact that said de-bubbling means (10) are placed around said one-way valve means (8).

7. Device (1) according to claim 6, **characterized by** the fact that said de-bubbling means (10) define a de-bubbling chamber (11), the walls of which are permeable to air and liquids, said one-way valve means (8) and said fourth port (7a) being placed inside said de-bubbling chamber (11).

8. Device (1) according to one or more of the preceding claims, **characterized by** the fact that said containment volume (3) has a maximum cross-section, transversely to the longitudinal extension of said ducts (4, 5, 6, 7), in the proximity of said opening (9a).

9. Device (1) according to claim 8, **characterized by** the fact that said bag (2) comprises at least a first lateral edge (3a), delimiting at least part of said containment volume (3) and defining a concavity (13) facing inwards from the containment volume itself.

10. Device (1) according to claim 9, **characterized by** the fact that said first lateral edge (3a) has an at least partly curved extension.

11. Device (1) according to one or more of the preceding claims, **characterized by** the fact that said first duct (4), second duct (5) and third duct (6) are placed, respectively, at a first zone (14), at a second zone (15) and at a third zone (16) of said bag (2), where said first zone (14) is placed in the upper portion of said bag (2) and where said second zone (15) and third zone (16) are placed in the lower portion of said bag (2).

12. Device (1) according to claim 9 and claim 11, **characterized by** the fact that said first lateral edge (3a) connects said first zone (14) to said third zone (16).

## Patentansprüche

1. Vorrichtung (1) zum Vorfüllen eines extrakorporalen Kreislaufs, umfassend:
- einen Beutel (2) aus flexiblem Material, der ein Aufnahmevolumen (3) für eine Flüssigkeit definiert;
und mindestens drei Leitungen, die verwendet werden als:
- mindestens eine erste Leitung (4), die mit dem Beutel (2) verbunden und mit einem ersten Durchgangsanschluss (4a) versehen ist, die im Inneren des Aufnahmevolumens (3) angeordnet ist, um eine Betriebsflüssigkeit in dieses einzuführen;
- mindestens eine zweite Leitung (5), die mit dem Beutel (2) verbunden und mit einem zweiten Durchgangsanschluss (5a) versehen ist, die im Inneren des Aufnahmevolumens (3) angeordnet ist, um die Betriebsflüssigkeit nach außen abfließen zu lassen;
- mindestens eine dritte Leitung (6), die mit dem Beutel (2) verbunden und mit einem dritten Durchgangsanschluss (6a) versehen ist, die im Inneren des Aufnahmevolumens (3) angeordnet ist, um die Betriebsflüssigkeit wieder in dieses einzuführen;
- mindestens eine vierte Leitung (7), die mit dem Beutel (2) verbunden und mit einem vierten Durchgangsanschluss (7a) versehen ist, die im Inneren des Aufnahmevolumens (3) angeordnet ist, um die im Aufnahmevolumen selbst und in der Betriebsflüssigkeit vorhandene Luft nach außen abzuleiten;
wobei die erste, zweite, dritte und vierte Leitung (4, 5, 6, 7) voneinander getrennt sind, oder die erste Leitung (4) mit der zweiten Leitung (5) zusammenfällt, der erste Anschluss (4a) mit dem zweiten Anschluss (5a) zusammenfällt,
oder die erste Leitung (4) ist von der zweiten Leitung (5) getrennt ist und mit der vierten Leitung (7) zusammenfällt, wobei der erste Anschluss (4a) mit dem vierten Anschluss (7a) zusammenfällt;
**dadurch gekennzeichnet, dass** sie Einwegventilmittel (8) umfasst, die mit der dritten Leitung (6) an der Stelle verbunden sind, an der sich der dritte Anschluss (6a) befindet, und die dazu ausgebildet sind, den Durchgang der Betriebsflüssigkeit von dem Aufnahmevolumen (3) zu der dritten Leitung (6) zu verhindern, und dadurch, dass die Einwegventilmittel (8) mindestens eine Aufnahmekammer (9) des dritten Anschlusses (6a) definieren, wobei die Aufnahmekammer (9) mit mindestens einer verschließbaren Öffnung (9a) versehen ist, die dazu ausgebildet ist, den Durchgang der aus dem dritten Anschluss (6a) fließenden Betriebsflüssigkeit innerhalb des Aufnahmevolumens (3) zu ermöglichen.

2. Vorrichtung (1) nach Anspruch 1, **dadurch gekennzeichnet, dass** die Einwegventilmittel (8) von der Art eines Beutels aus flexiblem Material sind, der die Aufnahmekammer (9) und die verschließbare Öffnung (9a) definiert.

3. Vorrichtung (1) nach Anspruch 1 oder 2, **dadurch gekennzeichnet, dass** die Einwegventilmittel (8) mindestens zwei Lagen aus flexiblem Material umfassen, die entlang mindestens drei Seiten miteinander abgedichtet sind.

4. Vorrichtung (1) nach einem der Ansprüche 1 bis 3, **dadurch gekennzeichnet, dass** die verschließbare Öffnung (9a) im Wesentlichen dem dritten Anschluss (6a) zugewandt ist.

5. Vorrichtung (1) nach einem oder mehreren der vorhergehenden Ansprüche, **dadurch gekennzeichnet, dass** sie Mittel zur Entfernung von Blasen (10) für die in der Betriebsflüssigkeit enthaltene Luft umfasst, die in dem Aufnahmevolumen (3) angeordnet sind.

6. Vorrichtung (1) nach Anspruch 5, **gekennzeichnet durch** die Tatsache, dass die Mittel zur Entfernung von Blasen (10) um die Einwegventilmittel (8) herum angeordnet sind.

7. Vorrichtung (1) nach Anspruch 6, **dadurch gekennzeichnet, dass** die Mittel zur Entfernung von Blasen (10) eine Blasenentfernungskammer (11) definieren, deren Wände für Luft und Flüssigkeiten durchlässig sind, wobei die Einwegventilmittel (8) und der vierte Anschluss (7a) innerhalb der Blasenentfernungskammer (11) angeordnet sind.

8. Vorrichtung (1) nach einem oder mehreren der vorhergehenden Ansprüche, **dadurch gekennzeichnet, dass** das Aufnahmevolumen (3) einen maximalen Querschnitt quer zur Längsausdehnung der Leitungen (4, 5, 6, 7) in der Nähe der Öffnung (9a) aufweist.

9. Vorrichtung (1) nach Anspruch 8, **dadurch gekennzeichnet, dass** der Beutel (2) mindestens eine erste seitliche Kante (3a) aufweist, die mindestens einen Teil des Aufnahmevolumens (3) begrenzt und eine Aushöhlung (13) definiert, die vom Aufnahmevolumen selbst nach innen weist.

10. Vorrichtung (1) nach Anspruch 9, **dadurch gekennzeichnet, dass** die erste seitliche Kante (3a) eine zumindest teilweise gekrümmte Ausdehnung aufweist.

11. Vorrichtung (1) nach einem oder mehreren der vorhergehenden Ansprüche, **dadurch gekennzeichnet, dass** die erste Leitung (4), die zweite Leitung (5) und die dritte Leitung (6) jeweils in einer ersten Zone (14), einer zweiten Zone (15) bzw. einer dritten Zone (16) des Beutels (2) angeordnet sind, wobei die erste Zone (14) im oberen Abschnitt des Beutels (2) angeordnet ist und die zweite Zone (15) und die dritte Zone (16) im unteren Abschnitt des Beutels (2) angeordnet sind.

12. Vorrichtung (1) nach Anspruch 9 und Anspruch 11, **dadurch gekennzeichnet, dass** die erste seitliche Kante (3a) die erste Zone (14) mit der dritten Zone (16) verbindet.

## Revendications

1. - Dispositif (1) pour l'amorçage d'un circuit extracorporel, comprenant :
- un sac (2) réalisé en matériau souple et définissant un volume de confinement (3) d'un liquide ;
et au moins trois conduits utilisés comme :
- au moins un premier conduit (4) associé audit sac (2) et comportant un premier orifice de transit (4a) placé à l'intérieur dudit volume de confinement (3) pour l'introduction d'un liquide de travail à l'intérieur de celui-ci ;
- au moins un deuxième conduit (5) associé audit sac (2) et comportant un deuxième orifice de transit (5a) placé à l'intérieur dudit volume de confinement (3) pour l'écoulement du liquide de travail vers l'extérieur ;
- au moins un troisième conduit (6) associé audit sac (2) et comportant un troisième orifice de transit (6a) placé à l'intérieur dudit volume de confinement (3) pour la réintroduction du liquide de travail à l'intérieur de celui-ci ;
- au moins un quatrième conduit (7) associé audit sac (2) et comportant un quatrième orifice de transit (7a) placé à l'intérieur dudit volume de confinement (3) pour l'écoulement vers l'extérieur de l'air présent dans le volume de confinement lui-même et dans le liquide de travail ;
dans lequel les premier, deuxième, troisième et quatrième conduits (4, 5, 6, 7) sont séparés les uns des autres, ou
ledit premier conduit (4) coïncide avec ledit deuxième conduit (5), ledit premier orifice (4a) coïncidant avec ledit deuxième orifice (5a), ou
ledit premier conduit (4) est séparé dudit deuxième conduit (5) et coïncide avec ledit quatrième conduit (7), ledit premier orifice (4a) coïncidant avec ledit quatrième orifice (7a) ;
**caractérisé par le fait qu'**il comprend des moyens de soupape unidirectionnelle (8) associés audit troisième conduit (6), à l'endroit où se trouve ledit troisième orifice (6a), et aptes à empêcher le transit du liquide de travail dudit volume de confinement (3) audit troisième conduit (6), et **par le fait que** lesdits moyens de soupape unidirectionnelle (8) définissent au moins une chambre de confinement (9) dudit troisième orifice (6a), ladite chambre de confinement (9) comportant au moins une ouverture pouvant être fermée (9a), apte à permettre le transit du liquide de travail s'écoulant hors dudit troisième orifice (6a) à l'intérieur dudit volume de confinement (3).

2. - Dispositif (1) selon la revendication 1, **caractérisé par le fait que** lesdits moyens de soupape unidirectionnelle (8) sont du type d'un sac réalisé en matériau souple définissant ladite chambre de confinement (9) et ladite ouverture pouvant être fermée (9a).

3. - Dispositif (1) selon la revendication 1 ou 2, **caractérisé par le fait que** lesdits moyens de soupape unidirectionnelle (8) comprennent au moins deux feuilles réalisées en matériau souple et scellées ensemble le long d'au moins trois côtés.

4. - Dispositif (1) selon les revendications 1 à 3, **caractérisé par le fait que** ladite ouverture pouvant être fermée (9a) est sensiblement en regard dudit troisième orifice (6a).

5. - Dispositif (1) selon l'une ou plusieurs des revendications précédentes, **caractérisé par le fait qu'**il comprend des moyens de débullage (10) de l'air contenu dans le liquide de travail placé à l'intérieur dudit volume de confinement (3).

6. - Dispositif (1) selon la revendication 5, **caractérisé par le fait que** lesdits moyens de débullage (10) sont placés autour desdits moyens de soupape unidirectionnelle (8).

7. - Dispositif (1) selon la revendication 6, **caractérisé par le fait que** lesdits moyens de débullage (10) définissent une chambre de débullage (11) dont les parois sont perméables à l'air et aux liquides, lesdits moyens de soupape unidirectionnelle (8) et ledit quatrième orifice (7a) étant placés à l'intérieur de ladite chambre de débullage (11).

8. - Dispositif (1) selon l'une ou plusieurs des revendications précédentes, **caractérisé par le fait que** ledit volume de confinement (3) a une section transversale maximale, transversalement à l'étendue longitudinale desdits conduits (4, 5, 6, 7), à proximité de ladite ouverture (9a).

9. - Dispositif (1) selon la revendication 8, **caractérisé par le fait que** ledit sac (2) comprend au moins un premier bord latéral (3a), délimitant au moins une partie dudit volume de confinement (3) et définissant une concavité (13) tournée vers l'intérieur à partir du volume de confinement lui-même.

10. - Dispositif (1) selon la revendication 9, **caractérisé par le fait que** ledit premier bord latéral (3a) a une extension au moins partiellement incurvée.

11. - Dispositif (1) selon l'une ou plusieurs des revendications précédentes, **caractérisé par le fait que** lesdits premier conduit (4), deuxième conduit (5) et troisième conduit (6) sont placés, respectivement, à une première zone (14), à une deuxième zone (15) et à une troisième zone (16) dudit sac (2), où ladite première zone (14) est placée dans la partie supérieure dudit sac (2) et où lesdites deuxième zone (15) et troisième zone (16) sont placées dans la partie inférieure dudit sac (2).

12. - Dispositif (1) selon la revendication 9 et la revendication 11, **caractérisé par le fait que** ledit premier bord latéral (3a) relie ladite première zone (14) à ladite troisième zone (16).
